# EUROPÄISCHE PATENTSCHRIFT

(11) **EP 0 254 289 B1**
(45) Veröffentlichungstag und Bekanntmachung des Hinweises auf die Patenterteilung: **20.10.1993**
(21) Anmeldenummer: 87110603.5
(22) Anmeldetag: 22.07.1987
(51) Int. Cl.: A61K 37/18, A61K 9/00

(54) **Mittel zur Behandlung von Arthrosen**
Agent for the treatment of arthrosis
Agent pour le traitement d'arthroses

(30) Priorität: 25.07.1986 DE 3625185
(43) Veröffentlichungstag der Anmeldung: 27.01.1988
(73) Patentinhaber: Deutsche Gelatine-Fabriken Stoess AG, 69402 Eberbach (DE)
(72) Erfinder: Koepff, Peter, Dr., D-6900 Heidelberg (DE); Müller, Alexander, D-6930 Eberbach (DE); Schrieber, Reinhard, D-6930 Eberbach (DE); Turowski, Angelika, Dr., D-6930 Eberbach (DE); Bräumer, Klaus, Dr., D-6930 Eberbach (DE)
(74) Vertreter: Werner, Hans-Karsten, Dr.Dipl.-Chem.

(56) Entgegenhaltungen:
- CH-A- 494 250
- DE-A- 2 462 222
- ERFAHRUNGSHEILKUNDE, HEIDELBERG, Band 28, Nr. 11, 1979, Seiten 930-938; E. KRUG: "Zur unterstützenden Therapie bei Osteo- und Chondropathien"

## Beschreibung

Gegenstand der vorliegenden Erfindung sind Mittel zur Behandlung von Arthrosen enthaltend geschmackloses oder geschmacksneutrales, enzymatisch hydrolysiertes Kollagen mit einem mittleren Molekulargewicht von 10 bis 80 KD. Weiterhin betrifft die Erfindung die Verwendung dieser Kollagene zur Herstellung von Mitteln zur Behandlung von Arthrosen.

Degenerative Gelenkerkrankungen, üblicherweise als Arthrosen bekannt, finden sich vorzugsweise im bradythrophen Gewebe (Sehnen, Knorpel), welches bekanntermaßen schlecht oder gar nicht durchblutet wird und aufgrund seines langsamen Stoffwechsels auf äußere Noxen weder mit entzündlichen noch mit regenerativen Prozessen reagieren kann. Es handelt sich dabei um ein Leiden, welches gehäuft im älteren Bevölkerungsanteil vorkommt, jedoch nur deshalb, weil das Durchschnittsalter der Bevölkerung sich erhöht hat und somit mehr Menschen ihre Arthrose erleben.

Zur Pathogenese der Arthrose läßt sich jedoch sagen, daß es sich dabei keineswegs um eine unvermeidliche Alterserscheinung handelt, da ein normales Gelenk bis ins hohe Alter funktionsfähig ist. In der Wissenschaft werden deshalb sogenannte primäre von sekundären Arthrosen unterschieden, wobei letztere auf dem Boden angeborener oder erworbener Gelenkschäden, also bei bekannten Vorerkrankungen sich entwickeln, während bei primären Arthrosen keine Grunderkrankung eruierbar ist. Vielmehr handelt es sich bei diesen Erkrankungen um ein Mißverhältnis zwischen mechanischer Beanspruchung und mechanischer Resistenz des Gelenkknorpels.

Prädilektionsstellen bilden in diesen Fällen die gewichtstragenden Gelenke, das sind Hüft- und Kniegelenke. In diesen Gelenken wird der knorpelige Überzug der gelenkbildenden Anteile in der Belastungszone aufgebraucht, so daß der subchondrale Knochen freiliegt und die blanken Knochenflächen aufeinanderreiben. Dies ist ein langsam fortschreitender progredienter Prozeß, wobei zunächst keine, später jedoch, wenn der schützende Knorpel aufgebraucht ist, erhebliche Beschwerden auftauchen.

Die konservativen Behandlungsmaßnahmen bei Coxarthrosen bzw. Gonarthrosen bis hin zu den operativen Maßnahmen wie künstlicher Gelenkersatz sind hinreichend bekannt.

Aus empirischen und klinischen Studien ergab sich die Andeutung, daß gelatinehaltige Präparate bei der Therapie der generativen Gelenkerkrankung eine unterstützende Rolle spielen können (Erfahrungsheilkunde, Heidelberg, 1979, Band 28, Nr. 11, S. 930 938, E. Krug).

CH No. 494 250 beschreibt ein Verfahren zur Herstellung von Gelatine aus hydrolisierten Kollagenen durch alkalische oder saure Hydrolyse sowie ihre Verwendung als Arzneimittel. Weiterhin beschreibt die Patentschrift, daß klinische Versuche mit hydrolisierten Kollagenen entsprechend Beispiel 8 dieser Schrift gegen Arthrose erfolgreich angewendet werden konnten.

DE-OS 24 62 222 beschreibt ein, Verfahren zur Herstellung von löslichem nativem Kollagen, dessen nicht-helikale Peptide teilweise oder vollständig abgetrennt sind. Das Kollagen kann im medizinischen Bereich zur Heilung von Störungen oder Erkrankungen der Gelenkhaut an Knochen, Knorpeln und Senen sowie zum Heilen von Rheumatismus verwendet werden.

Gelatine ist ein denaturiertes Kollagen, welches nur in heißem Wasser löslich ist und beim Abkühlen in der Lage ist, erhebliche Mengen Wasser zu binden. Sie wird aus diesem Grund bei einer Reihe von Lebensmitteln und Arzneimitteln eingesetzt, jedoch nicht als Wirkstoff. Die Einnahme von größeren Mengen Gelatine ist schwierig und unangenehm und führt darüber hinaus zu Völlegefühl und Übelkeit.

Die vorliegende Erfindung hat sich die Aufgabe gestellt, Mittel zur Behandlung von Arthrosen zur Verfügung zu stellen, welche möglichst lindernd und heilend bei Arthrosen wirken, dabei jedoch gut verträglich und gut einnehmbar sind.

Diese Aufgabe wird durch Mittel zur Behandlung von Arthrosen enthaltend geschmackloses oder geschmacksneutrales, enzymatisch hydrolysiertes Kollagen aus tierischer Haut, tierischen Knochen, ausreichend gereinigtem Bindegewebe oder Gelatine mit einem mittleren Molekulargewicht von 10 bis 80 KD gelöst.

Intensive klinische Untersuchungen haben jetzt zu dem überraschenden Ergebnis geführt, daß kaltwasserlösliche Peptide, inbesondere aus der Gruppe der hydrolysierten Kollagene, tatsächlich zur Behandlung von Arthrosen geeignet sind. Besonders geeignet ist enzymatisch hydrolysiertes Kollagen mit einem mittleren Molekulargewicht von 10 bis 80 KD. Diese hydrolysierten Peptide lassen sich in Form von Pasten, Sirupen, Lösungen, Granulaten, Komprimaten oder instantisiertem Pulver gut einnehmen. Da sie nahezu geschmacklos bzw. geschmacksneutral sind, lassen sie sich besonders gut zusammen mit Geschmacksstoffen, Süßungsmitteln und/oder Aromastoffen einnehmen. Da sich für eine wirksame Therapie Tagesdosen von 5 bis 12 g als besonders wirksam erwiesen haben, empfiehlt es sich, die hydrolysierten Kollagene in Dosisformen abzupacken, welche 0,5 bis 12 g hydrolysiertes Kollagen enthalten.

Bei den vergleichenden klinischen Untersuchungen wurde festgestellt, daß eine besonders gute Verträglichkeit und Einnehmbarkeit einerseits und eine besonders gute Wirksamkeit gegen Arthrosen andererseits beobachtet wird bei hydrolysierten Kollagenen mit einem mittleren Molekulargewicht von 10 bis 80 KD. Höhermolekulare Präparate sind meistens nicht mehr schnell und leicht genug in kaltem Wasser löslich. Niedermolekulare Produkte sind schwieriger in akzeptable Applikationsformen zu bringen. Proteine, die nicht aus Kollagen gewonnen wurden, haben sich als unwirksam oder wesentlich schwächer wirksam erwiesen.

Kollagen ist ein Faserprotein, welches den Hauptbestandteil des Stütz- und Bindegewebes bei Tier und Mensch bildet und sich insbesondere in der Haut, den Sehnen und Knochen findet.

Hydrolysiertes Kollagen, insbesondere enzymatisch hydrolysiertes Kollagen, kann daher sowohl aus tierischer Haut, tierischen Knochen sowie sonstigem ausreichend gereinigten Bindegewebe hergestellt werden. Es ist weiterhin prinzipiell möglich, auch bereits denaturiertes Kollagen (Gelatine) als Rohstoff einzusetzen.

Die Hydrolyse wird durch enzymatische Hydrolyse durchgeführt. Bei jedem Abbau können die Hydrolysebedingungen so gewählt werden, daß ein bestimmter Molekulargewichtsbereich erhalten wird. Je nach Herstellungsbedingungen weisen die Hydrolysate mehr Carboxylgruppen oder mehr Aminogruppen auf und besitzen einen verschiedenen isoelektrischen pH-Bereich. Besonders schonend ist die enzymatische Hydrolyse, bei der besonders reine und salzarme Produkte entstehen.

Derartige enzymatisch hydrolysierte Kollagene werden beispielsweise von der Anmelderin hergestellt und unter der Bezeichnung Gelita-Sol vertrieben. Diese enzymatisch hydrolysierten Kollagene weisen meistens ein mittleres Molekulargewicht von 30 bis 45 KD auf. Die Molekulargewichtsverteilung reicht von 2 bis 80 KD. Die Hauptmenge liegt jedoch im Bereich zwischen 5 und 40 KD. Diese enzymatisch hydrolysierten Kollagene sind mehr oder weniger geschmacklos oder zumindest geschmacksneutral. Sie sind in kaltem Wasser löslich und vermögen nicht mehr wie denaturiertes Kollagen (Gelatine) erhebliche Wassermengen zu binden. Sie sind jedoch gut dispergierbar und emulsionsstabilisierend. Sie wirken auch schaumstabilisierend und cremestabilisierend. Sie können filmbildend sein und Klebeeigenschaften aufweisen.

Mit Tensiden neigen sie zu Komplexbildung und verbessern dadurch die Hautverträglichkeit von Tensiden. Sie verbessern die Trocken- und Nasskämmbarkeit und den Glanz des Haares. Sie unterstützen die Verdauungstätigkeit und sind geeignet, Vitamine und Aromen zu umhüllen bzw. einzubetten. Schließlich werden sie verwendet zur Verbesserung der Absorption von Färbemitteln auf Haaren und Wolltextilien. In der Diätetik wurden sie bisher verwendet als allgemeine Schonkost bei Magen- und Darmerkrankungen, Verdauungsstörungen, Reduktionskost, Diabetes oder auch als Aufbaukost für Sportler. Entscheidend war dabei die leichte Verdaulichkeit des Eiweißes, die Schutzkolloidwirkung, die eine Feinverteilung der Nährstoffe bewirkt und damit eine Verbesserung der Gesamtverdaulichkeit der Speisen, ein gutes Bindevermögen, durch welches sich Speisen ohne Fett zubereiten lassen, das Fehlen von Kohlenhydraten sowie die Purinfreiheit.

Im Lebensmittelbereich wurden sie eingesetzt in der Fleischwarenindustrie, in der Süßwaren- und Getränkeindustrie. In der Pharmazie wurden sie eingesetzt als Tablettierhilfsmittel, Umhüllungsagenz und Füllmittel. Durch die jetzt durchgeführten intensivien klinischen Untersuchungen und Doppelblindstudien hat sich herausgestellt, daß insbesondere diese enzymatisch hydrolysierten Kollagene in Mengen von 5 bis 12 g pro Tag lindernd und heilend bei Arthrosen wirken. Besonders überraschend war dabei der Befund, daß bereits während dieser Therapie eine analgetische Wirkung beobachtet wurde, die sich in einem verminderten Bedarf sonstiger Analgetika deutlich bemerkbar machte.

Gegenstand der vorliegenden Erfindung sind somit zunächst Mittel zur Behandlung von Arthrosen enthaltend hydrolysiertes Kollagen, wobei enzymatisch hydrolysiertes Kollagen mit einem mittleren Molekulargewicht Von 10 bis 80 KD besonders bevorzugt ist.

Weiterhin betrifft die Erfindung die Verwendung von diesen enzymatisch hydrolisierten Kollagenen zur Herstellung von Mitteln zur Behandlung von Arthrosen.

In den nachfolgenden Beispielen sind die erfindungsgemäßen Mittel, ihre Herstellung und ihre Anwendung näher erläutert.

### Beispiel 1

Enzymatisch hydrolysiertes Kollagen wurde hergestellt durch Schneiden, Waschen und enzymatische Hydrolyse von frischem Rinderspalt oder frischer Schweinehaut im Rührtank, Inaktivierung des Enzyms, Abtrennung, Filtration, Aufkonzentrierung, Sterilisation und Sprühtrocknung. Es handelt sich um hellgelbe bis weißliche Pulver, die kaltwasserlöslich sind, geschmacksneutral bzw. nahezu geschmacklos sind und Korngrößen unter 0,5 mm aufweisen. Es handelte sich um die Handelsprodukte der Anmelderin Gelita-Sol D (Diätetik) und P (Pharmazie). Das mittlere Molekulargewicht betrug 40 KD bzw. 60 KD.

Beide Pulver lassen sich nach Anfeuchten mit etwas Wasser zu Tabletten komprimieren, die sich sowohl in Wasser wie auch im Magensaft rasch wieder auflösen.

Zur leichteren Einnehmbarkeit wurden Tabletten von je 0,5 g hergestellt, von denen 10 bis 24 Stück über den Tag verteilt nach Belieben eingenommen werden können.

Größere Mengen können auch appliziert werden in Form von Pasten, Sirup, Lösungen, Granulaten, Komprimaten oder instantisierten Pulvern, wobei auch fertige Instantgetränke oder Instantsuppen in Frage kommen.

### Beispiel 2

Folgende Doppelblindstudie wurde durchgeführt: Tabletten enthaltend 0,5 g enzymatisch hydrolysiertes Kollagen gemäß Beispiel 1 wurden im Vergleich zu entsprechenden Tabletten enthaltend 0,5 g Gelatinepulver oder 0,5 g Protein aus Hühnereiweiß eingesetzt. Es wurden 81 Patienten untersucht, wobei 29 aus unterschiedlichen Gründen die Behandlung abbrechen mußten. Die verbleibenden 52 Patienten (24 Frauen, 28 Männer) sind für die vorliegende Studie relevant.

Bei diesem Patientenkollektiv handelte es sich um Erkrankungen des degenerativen Formenkreises des Hüftund/oder des Kniegelenkes, wobei 10 Probanden eine einseitige Coxarthrose und 31 Probanden degenerative Veränderungen an beiden Hüftgelenken aufwiesen. Bei 21 Patienten lag eine ausgeprägte Arthrose der Kniegelenke vor, in 6 Fällen waren beide Kniegelenke betroffen. Degenerative Gelenkserkrankungen des Hüft- und Kniegelenkes fanden sich bei 10 Probanden. Die Krankheit dauerte bei mehr als der Hälfte der Probanden bereits über 5 Jahre und nur bei etwa 10% war sie kürzer als 2 Jahre.

Die Diagnosestellung erfolgte mit Hilfe röntgenologischer Kriterien, wobei es sich um Osteoarthrosen vom ersten bis zum dritten Stadium handelte. Wie in Tabelle 1 aufgezeigt, lagen bei allen 52 Patienten Gelenkspaltverschmälerungen vor. Klinisch handelte es sich in allen Fällen um eine aktivierte Form der Arthrose. Eine subchondrale Sklerosierung lag in 28 Fällen vor. Osteophyten fanden sich bei 41, Zysten bei 10 und Erosionen bei 3 Patienten. Sechs Patienten wiesen bereits eine Protrusio acetabuli auf.

In der klinischen Studie wurden folgende subjektive Beschwerden als Kriterien für die Beurteilung der getesteten Substanzen mit einbezogen:
- Anlaufschmerz
- Anlaufsteifheit
- Kraftlosigkeit
- Kälteempfindlichkeit
- Belastungsschmerz
- Ermüdungsschmerz
- Muskelschmerz
- Nachtschmerz
- Wetterempfindlichkeit
- Druckdolenz über dem Gelenkspalt
- Druckdolenz am Trochanter
- Druckdolenz an Sehnenansätzen
- Bewegungsschmerz
- Endphasenschmerz

Die Beweglichkeit der betroffenen Gelenke wurde wie üblicherweise durch Ausmessen des Winkels zwischen Grund- und Endposition in allen physiologischen Ebenen überprüft. Die durchschnittliche Beweglichkeit aller untersuchten Gelenke ist aus Tabelle 2 ersichtlich.

Die in Tabelle 2 aufgeführten subjektiven Kriterien wurden in einem Zweistufentest gemäß den Angaben des Patienten in mäßig (1 Punkt) bzw. deutlich ausgeprägt (2 Punkte) beurteilt. Vom so erhaltenen Ausgangsscore wurde der nach Ablauf eines Behandlungszyklus einer jeden Testsubstanz neu ermittelte Score abgezogen.

Darüber hinaus wurden folgende Laboruntersuchungen durchgeführt:
- Blutsenkungsgeschwindigkeit nach einer und zwei Stunden
- Transaminasen
- Alkalische und saure Phosphatasen
- Antikörper im Serum gegen Typ I-, Typ II-, Typ III-Kollagen

### Testsubstanzen

Es kamen folgende Substanzen zum Einsatz:
Testsubstanz Nr. 173 - Gelita-Sol D (erfindungsgemäß)
Testsubstanz Nr. 174 (Reference-Substanz) - Protein aus Hühnereiweiß
Testsubstanz Nr. 175 (Reference-Substanz) - Gelatinepulver (Spezialqualität für Diätzwecke)

Jede der drei angegebenen Substanzen wurde in beliebiger Reihenfolge über jeweils 60 Tage appliziert, wobei ein behandlungsfreies Intervall von ca. 2 Monaten vor der nächstfolgenden Einnahme lag. Verabreicht wurden 10 g Substanz pro Tag in Form von 20 Stück 0,5 g schweren Tabletten, die von den Patienten über den Tag verteilt nach Belieben eingenommen werden konnten.

Auf Antiarthrotika (z.B. Arumalon oder Arteparon) wurde während des Untersuchungszeitraumes bewußt verzichtet, wohingegen Analgetika unter gleichzeitiger Dosiskontrolle weiter eingenommen werden durften (siehe Tabelle 4).

### Ergebnisse

Die Untersuchungen wurden mit einem Patientenkollektiv von 81 Personen begonnen, wobei 29 Patienten die Behandlung aus unterschiedlichen Gründen vorzeitig abbrachen. Sechs Patienten gaben bei Präparat 175 einen unangenehmen Druck im Magen an, acht fühlten sich so gut, daß sie eine weitere Einnahme der Testsubstanzen ablehnten und bei den restlichen Patienten (15) traten Noxen so unterschiedlicher Art wie Herzschlag, Hepatitis, Karzinom auf. Ein Zusammenhang mit der Verabreichung der Testsubstanzen ist hier nicht zu erkennen.

Bei den verbleibenden 52 Patienten zeigten sich folgende Ergebnisse:

### Transaminase/Phosphatase

Der Blutspiegel der Probanden war normal und blieb auch während der gesamten Untersuchungszeit konstant.

### Blutsenkungsgeschwindigkeit

Die BSG-Werte waren zu Beginn des Versuches normal bis leicht erhöht, sie lagen durchschnittlich bei 11/1 Stunde und 24/2 Stunden. Die höchste Beschleunigung lag bei 20/42. Während der Versuchsdauer wurde keine relevante Änderung der Werte festgestellt.

### Antikörper

Die Mittelwerte der Gehalte an Antikörpern im Serum gegen Kollagen betrugen:
Gegen Typ I - 4,357 (-log₂)
Gegen Typ II - 3,642 (-log₂)
Gegen Typ III - 4,07 (-log₂)

Der Antikörper-Titer veränderte sich während der Verabreichung der Testsubstanzen nur unwesentlich.

### Auswertung der Befunde

Nach Abschluß der Versuche wurde für jede Testsubstanz der Score berechnet und mit dem Anfangsscore verglichen. Wie aus den Tabellen 3 und 4 ersichtlich, wurden zwar die besten Ergebnisse mit der Testsubstanz Nr. 175 erreicht, jedoch führte dieses Präparat häufig zur "Nebenwirkung" Völlegefühl. Bei 30 Patienten war der Endscore nach Verabreichung von Nr. 175 um mehr als 50% verringert. Nur bei 10 Probanden betrug die Verringerung des Anfangsscores 25% oder weniger. Die Testsubstanz Nr. 173 zeigte im Prinzip sehr ähnliche Werte. Hingegen waren die Unterschiede zur Substanz Nr. 174 signifikant. Mit dieser Substanz ließ sich nur ein unwesentlicher Einfluß auf das Krankheitsbild feststellen, wie auch aus der geringfügigen Erniedrigung des Ausgangsscores ersichtlich, welcher nämlich bei 40 Patienten nicht mehr als 25% betrug.

Die 12 Patienten, bei denen die Verringerung des Ausgangsscores über 25% lag, hatten vor der Verabreichung von Nr. 174 schon die anderen beiden Substanzen des Versuchsprogrammes eingenommen.

Bei einem erneuten Versuch mit ausschließlicher Gabe des Testsubstanz Nr. 174 lag die Verringerung der Ausgangsscores bei allen Probanden ausnahmslos unter 25%.

Die Tabelle 4 enthält die Reduzierung der Analgetika-Verabreichung am Ende jedes Testzyklus mit der entsprechenden Testsubstanz, gemessen in Prozenten. Auch hier wie in Tabelle 3 schneiden die Testsubstanzen Nr. 173 und 175 am besten ab, wohingegen Nr. 174 keine so drastische Senkung des Analgetika-Bedarfs hervorrief.

Als positive marginale Beobachtungen konnten bei einigen Patienten während der Kur Gewichtsreduktionen festgestellt werden. Weiterhin wurde eine Linderung von Wirbelsäulebeschwerden sowie eine Verbesserung des allgemeinen Wohlbefindens angegeben, so daß manche Patienten sogar nach einer Fortführung der Therapie verlangten. Auch bei diesen Beobachtungen zeigte Testsubstanz Nr. 175 die signifikanteste Wirkung, dicht gefolgt von Nr. 173.

### Röntgenuntersuchungen

Röntgenologische Veränderungen konnten weder im positiven noch negativen Sinne festgestellt werden.

**Tabelle 1**

| Osteoarthrose | |
|---|---|
| Röntgenologische Kriterien bei 52 Probanden vor Beginn der Studie | |
| Gelenkspaltverschmälerung | 52 |
| Subchondrale Sklerosierung | 28 |
| Osteophytose | 41 |
| Zysten | 10 |
| Erosionen | 3 |
| Protrusio acetabuli | 6 |

**Tabelle 2**

| Kriterien vor der Verabreichung der Testsubstanzen | |
|---|---|
| | positive Befunde |
| - Anlaufschmerz | 44 |
| - Anlaufsteifheit | 45 |
| - Kraftlosigkeit | 21 |
| - Kälteempfindlichkeit | 20 |
| - Belastungsschmerz | 52 |
| - Ermüdungsschmerz | 52 |
| - Muskelschmerz | 34 |
| - Nachtschmerz | 24 |
| - Wetterempfindlichkeit | 24 |
| - Druckdolenz über dem Gelenkspalt | 48 |
| - Druckdolenz am Trochanter | 25 |
| - Druckdolenz an Sehnenansätzen | 29 |
| - Bewegungsschmerz | 17 |
| - Endphasenschmerz | 52 |

| Beweglichkeit der Hüftgelenke (durchschnittlich) | |
|---|---|
| Flexion | 72° |
| Abduktion | 16° |
| Innenrotation | 6° |
| Außenrotation | 16° |

| Beweglichkeit der Kniegelenke | |
|---|---|
| Flexion | 92° |

**Tabelle 3**

| Scoreveränderung nach Verabreichung der Testsubstanzen | | | |
|---|---|---|---|
| Testsubstanzen | Erniedrigung des Ausgangsscores um | | |
| | >50% | 25-50% | <25% |
| 173 | 25 | 17 | 10 |
| 174 (Reference) | 5 | 7 | 40 |
| 175 (Reference) | 30 | 15 | 7 |

**Tabelle 4**

| Verminderung der Analgetika-Verabreichung am Ende eines jeden Testzyklus mit der entsprechenden Testsubstanz | | | | |
|---|---|---|---|---|
| Testsubstanzen | >75% | 51-75% | 26-50% | <26% |
| 173 | 21 | 15 | 9 | 17 |
| 174 (Reference) | 4 | 14 | 23 | 11 |
| 175 (Reference) | 23 | 16 | 7 | 6 |

## Patentansprüche

1. Mittel zur Behandlung von Arthrosen enthaltend geschmackloses oder geschmacksneutrales, enzymatisch hydrolysiertes Kollagen aus tierischer Haut, tierischen Knochen, ausreichend gereinigtem Bindegewebe oder Gelatine mit einem mittleren Molekulargewicht von 10 bis 80 KD.

2. Mittel gemäß Anspruch 1, dadurch gekennzeichnet, daß sie als Paste, Sirup, Lösung, Granulat, Komprimat oder instantisiertes Pulver vorliegen.

3. Mittel gemäß einem der Ansprüche 1 oder 2, dadurch gekennzeichnet, daß sie Geschmacksstoffe, Süßungsmittel und/oder Aromastoffe enthalten.

4. Mittel gemäß einem der Ansprüche 1 bis 3, dadurch gekennzeichnet, daß sie in Dosisformen abgepackt sind, welche 0,5 bis 12 g hydrolysiertes Kollagen enthalten.

5. Verwendung von geschmacklosen oder geschmacksneutralen, enzymatisch hydrolysierten Kollagenen aus tierischer Haut, tierischen Knochen, ausreichend gereinigtem Bindegewebe oder Gelatine mit einem mittleren Molekulargewicht von 10 bis 80 KD zur Herstellung von Mitteln zur Behandlung von Arthrosen.

6. Verwendung gemäß Anspruch 5, dadurch gekennzeichnet, daß die kaltwasserlöslichen Kollagene als Paste, Sirup, Lösung, Granulat, Komprimat oder instantisiertes Pulver eingesetzt werden.

7. Verwendung gemäß einem der Ansprüche 5 oder 6, dadurch gekennzeichnet, daß die kaltwasserlöslichen Kollagene zusammen mit Geschmacksstoffen, Süßungsmitteln und/oder Aromastoffen eingesetzt werden.

8. Verwendung gemäß einem der Ansprüche 5 - 7, dadurch gekennzeichnet, daß die kaltwasserlöslichen Kollagene in Dosisformen abgepackt werden, welche 0,5 bis 12 g hydrolysiertes Kollagen enthalten.

## Claims

1. Agents for the treatment of arthroses containing flavourless or flavour-neutral, enzymatically hydrolyzed collagen from animal skins, animal bones, sufficiently refined connective tissue or gelatin having an average molecular weight of from 10 to 80 kD.

2. Agents according to claim 1, characterized in that they are in the form of pastes, syrups, solutions, granulate, compacts or instantized powder.

3. Agents according to one of claims 1 or 2, characterized in that they contain flavourings, sweeteners and/or aromatics.

4. Agents according to one of claims 1 to 3, characterized in that they are packaged in dosage forms containing from 0.5 to 12 g of hydrolyzed collagen.

5. Use of flavourless or flavour-neutral, enzymatically hydrolyzed collagens from animal skins, animal bones, sufficiently refined connective tissue or gelatin having an average molecular weight of from 10 to 80 kD for the preparation of for treating arthroses.

6. The use according to claim 5, characterized in that the collagens soluble in cold water are used in the form of pastes, syrups, solutions, granulate, compacts or instantized powder.

7. The use according to one of claims 5 or 6, characterized in that the collagens soluble in cold water are used together with flavorings, sweeteners and/or aromatics.

8. The use according to one of claims 5 to 7, characterized in that the collagens soluble in cold water are packaged in dosage forms containing from 0.5 to 12 g of hydrolyzed collagen.

## Revendications

1. Agents pour le traitement d'arthroses, comprenant du collagène insipide ou de goût neutre, hydrolisé de manière enzymatique, provenant de peau d'animaux, d'os d'animaux, de tissu conjonctif ou de gélatine suffisamment épurés, présentant une masse moléculaire moyenne comprise entre 10 et 80 KD.

2. Agents selon la revendication 1, caractérisés en ce qu'ils se présentent sous la forme de pâte, de sirop, de solution, de granulés, de comprimés ou de poudre instantanée.

3. Agents selon l'une quelconque des revendications 1 ou 2, caractérisés en ce qu'ils comprennent des agents de sapidité, des édulcorants et/ou des substances aromatiques.

4. Agents selon l'une quelconque des revendications 1 à 3, caractérisés en ce qu'ils sont emballés sous forme de doses, lesquelles comprennent 0,5 à 12 g de collagène hydrolysé.

5. Utilisation de collagènes insipides ou de goût neutre, hydrolysés de manière enzymatique, provenant de peau d'animaux, d'os d'animaux, de tissu conjonctif ou de gélatine suffisamment épuré présentant une masse moléculaire moyenne de 10 à 80 KD pour la fabrication d'agents pour le traitement d'arthroses.

6. Utilisation selon la revendication 5, caractérisée en ce que les collagènes solubles dans l'eau froide peuvent être utilisés sous forme de pâte, de sirop, de solution, de granulés, de comprimés ou de poudre instantanée.

7. Utilisation selon l'une quelconque des revendications 5 ou 6, caractérisée en ce que les collagènes solubles dans l'eau froide peuvent être utilisés avec des agents de sapidité, des édulcorants et/ou des substances aromatiques.

8. Utilisation selon l'une quelconque des revendications 5 à 7, caractérisée en ce que les collagènes sont emballés sous forme de doses, lesquelles comprennent 0,5 à 12 g de collagène hydrolysé.
